(19) Europäisches Patentamt
European Patent Office
Office européen des brevets

(11) EP 4 414 323 A1

(12) **EUROPEAN PATENT APPLICATION**
published in accordance with Art. 153(4) EPC

(43) Date of publication:
**14.08.2024 Bulletin 2024/33**

(21) Application number: **22877971.6**

(22) Date of filing: **09.10.2022**

(51) International Patent Classification (IPC):
***C01B 15/023** (2006.01)* ***C07C 231/02** (2006.01)*

(52) Cooperative Patent Classification (CPC):
**C01B 15/023; C07C 231/02**

(86) International application number:
**PCT/CN2022/123976**

(87) International publication number:
**WO 2023/056957 (13.04.2023 Gazette 2023/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **09.10.2021 CN 202111176363**
**09.10.2021 CN 202111176349**
**09.10.2021 CN 202111176239**
**09.10.2021 CN 202111176361**

(71) Applicants:
- **China Petroleum & Chemical Corporation Beijing 100728 (CN)**
- **Sinopec Dalian Research Institute of Petroleum and Petrochemicals Co., Ltd. Lushunkou District Dalian, Liaoning 116045 (CN)**

(72) Inventors:
- **BAI, Hongxin Dalian, Liaoning 116045 (CN)**
- **FANG, Xiangchen Dalian, Liaoning 116045 (CN)**
- **LIU, Quanjie Dalian, Liaoning 116045 (CN)**
- **JIA, Liming Dalian, Liaoning 116045 (CN)**

(74) Representative: **karo IP**
**karo IP Patentanwälte**
**Kahlhöfer Rößler Kreuels PartG mbB**
**Postfach 32 01 02**
**40416 Düsseldorf (DE)**

# (54) WORKING SOLUTION FOR PRODUCING HYDROGEN PEROXIDE BY MEANS OF ANTHRAQUINONE METHOD AND SOLVENT SYSTEM THEREOF

(57) A solvent system of a working solution for producing hydrogen peroxide through anthraquinone process comprises or consists of the following components, in parts by volume: A) 2-60 parts of an imide derivative represented by formula (I); B) 40-98 parts of an aromatic hydrocarbon, preferably $C_{9-10}$ aromatic hydrocarbon; C) 0-20 parts of trioctyl phosphate; and D) 0 to 20 parts of diisobutylcarbinol. The solvent system achieves good solubility of both anthraquinone and anthrahydroquinone, low intersolubility with water and stable physicochemical properties, meets the use requirements of the process according to anthraquinone process, can significantly improve the production efficiency of the process for producing hydrogen peroxide through anthraquinone process, and has good industrial application prospects.

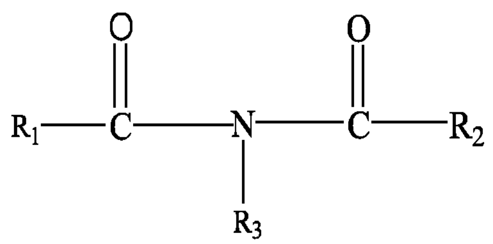

(I)

EP 4 414 323 A1

## Description

### Technical Field

The application relates to production of hydrogen peroxide, in particular to a working solution for producing hydrogen peroxide through anthraquinone process and a solvent system thereof.

### Background of Art

In recent years, green production processes such as preparation of propylene oxide by direct oxidation of propylene/hydrogen peroxide and preparation of caprolactam by ammoximation/oxidation of cyclohexanone have become increasingly established in China. The requirements on environmental protection are becoming increasingly strict. Hydrogen peroxide ($H_2O_2$) as a clean and environment-friendly chemical product is widely applied to the fields of chemical synthesis, environmental protection, papermaking bleaching, textile printing and dyeing, medical disinfection, electronics and the like. And the market demand is increased greatly year by year.

Currently, over 99% of hydrogen peroxide products in the world are all produced through anthraquinone process. In the process, an anthraquinone derivative dissolved in a solvent is hydrogenated in the presence of a catalyst to obtain a corresponding anthrahydroquinone compound, which is then oxidized to generate hydrogen peroxide; meanwhile, the anthrahydroquinone is oxidized back to the original anthraquinone; the generated hydrogen peroxide is extracted by pure water to obtain hydrogen peroxide products with different concentrations, and the raffinate can be recycled after being treated.

The working solution, as "blood" of anthraquinone process, has decisive impacts on the production efficiency of each production unit, and the industry generally uses the volume of $H_2O_2$ generated by single-pass conversion of unit volume of working solution (namely, the prepared $H_2O_2$ in gram per liter of working solution) as an important index for evaluating the production capacity. The solvent system of the ideal working solution should have the following characteristics: (1) the solvent having good chemical stability and relatively good hydrogenation resistance, oxidation resistance and hydrolysis resistance; (2) the solvent obtaining high solubility of both anthraquinone and anthrahydroquinone; (3) the difference between its density and the density of water being large, so that the working solution can be conveniently separated from water by extraction; (4) high interfacial tension, and $H_2O_2$ having a high distribution coefficient between $H_2O$ and the solvent; (5) low viscosity, high boiling point and flash point; (6) the solvent having low solubility in water and low solubility in $H_2O_2$ solution; (8) low toxicity. Due to the significant difference in molecular polarity between anthraquinone and its hydrogenated products, it is difficult for a single solvent to meet the above requirements for the working solution. The solvent system for the working solution is generally compounded by a non-polar solvent for anthraquinone and a polar solvent for anthrahydroquinone. Currently, $C_9$-$C_{10}$ heavy aromatic hydrocarbon (AR) is mainly used as the solvent for anthraquinone, and trioctyl phosphate (TOP) is mainly used as the solvent for anthrahydroquinone. However, the existing solvent system for the working solution mainly has the following defects: (1) The solubility of anthraquinone and anthrahydroquinone in the solvent system is relatively low, leading to a low conversion rate of anthraquinone, a large circulation volume of the working solution, and limited operational flexibility. Under normal temperature conditions, the solubility of 2-ethylanthraquinone in the AR/TOP solvent system is only 130-150 g/L, and the solubility of anthrahydroquinone under working conditions is only 55-60 g/L. In order to avoid the deterioration of the physicochemical properties of the working solution caused by the crystallization and precipitation of anthrahydroquinone, the conversion rate of anthraquinone is typically controlled at a relatively low level (usually below 40%) in industrial applications. The dissolving capacity of the working solution to anthraquinone and anthrahydroquinone jointly determines the maximum production capacity of the working solution for hydrogen peroxide. Insufficient dissolving capacity of the working solution is a bottleneck that limits the production efficiency. (2) The low hydrogenation efficiency of the working solution results in a low concentration of hydrogen peroxide products, limiting the production efficiency of plants and leading to higher production cost. Currently, the hydrogenation efficiency of domestic hydrogen peroxide industrial plants is generally lower than 8.5 g/L. Meeting the production load requirements of plants relies on a large circulation volume of the working solution within the reaction system, placing harsh requirements on the load of power equipment and the structure of the reactor. This not only poses significant engineering challenges but also results in high energy consumption, large investments, and relatively low concentrations of hydrogen peroxide products. Further purification and concentration are required to meet the quality concentration requirements of hydrogen peroxide for processes such as the production of propylene oxide (HPPO) through direct oxidation of propylene/hydrogen peroxide and the production of caprolactam through ammoximation of cyclohexanone and the like. If the hydrogenation efficiency of the working solution can be improved, it can not only significantly reduce the circulation volume of the working solution under the same working conditions but also eliminate the need for product purification and concentration procedures, thus reducing associated production costs and potential safety hazards. (3) The production process of hydrogen peroxide through anthraquinone process is a typical gas-liquid-solid three-phase reverse process. The existing working solution exhibits low hydrogen

dissolution and mass transfer capabilities, resulting in a low hydrogen content in the working solution. This further leads to a slower rate of anthraquinone hydrogenation reactions, poorer catalytic hydrogenation selectivity, and a higher degradation rate of anthraquinone, resulting in increased anthraquinone consumption and severely limiting the production efficiency of plants. (4) Severe degradation of the working solution leads to low-quality hydrogen peroxide products and high anthraquinone consumption, unable to meet the requirements for high-purity hydrogen peroxide products. The presence of degradation products in the working solution not only causes the loss of effective anthraquinone, increasing anthraquinone consumption, but also significantly deteriorates the physicochemical properties of the working solution. This results in poor fluidity, reduced interfacial tension, and a tendency for flooding to occur in the extraction tower. The working solution becomes difficult to condense in the extraction tower, leading to water carryover in the raffinate. The increased viscosity of the working solution and flow resistance affect the separation in the extraction tower. The increased density of the working solution reduces the density difference between the working solution and water, making it difficult to separate the two due to dissolution of the working solution together with water in the extraction tower. This further leads to a higher impurity content in the product. On the basis of an AR/TOP binary solvent system, Chinese patent application publication CN1552618A discloses a ternary solvent system of aromatic hydrocarbon + trioctyl phosphate + methylcyclohexyl acetate (AR/TOP/MCHA), which can increase the solubility of 2-ethyl anthraquinone in the AR/TOP/MCHA system by 30 g/L, and can increased the hydrogenation efficiency of the working solution to 9-9.5 g/L compared with the conventional AR/TOP binary system, but the working solution has high density and viscosity, the mass transfer capacity for hydrogen is low, and the hydrogenation conversion rate of anthraquinone under the working condition is nearly 40%.

Chinese patent application publication CN1583546A discloses a ternary solvent system of aromatic hydrocarbon + trioctyl phosphate + tetrabutyl urea (AR/TOP/TBU), which increases the solubility of anthrahydroquinone by about 10%, and then slightly improves the hydrogenation efficiency of the working solution compared with the conventional AR/TOP binary system. The working solution has suitable physical and chemical indexes such as density, viscosity and the like, but the system has relatively high intersolubility with water.

European patent application EP0287421 discloses a binary solvent system of aromatic hydrocarbon + N-phenyl N-ethyl benzamide (AR/BEA), which has significantly increased solubility of anthraquinone and anthrahydroquinone, but the working solution formed with anthraquinone has higher density and high water solubility, resulting in poor hydrogen peroxide extraction and separation effect, higher carbon residue in hydrogen peroxide products, and poor application effect, and thus cannot meet the requirements of industrial application.

Chinese patent application publication CN111071993A discloses a binary solvent system of aromatic hydrocarbon + tetraalkyl bisamide derivative, which can remarkably improve the dissolution and mass transfer of hydrogen in the working solution, can increase the solubility of 2-ethyl anthraquinone to 180 g/L or above, can increase the hydrogenation efficiency to 13g/L or above, and can meet the requirements of industrial application. However, the synthesis process of the tetraalkyl bisamide derivative molecule is complex, and the production cost is relatively high. These influence the industrial application and popularization of the working solution system.

Therefore, there is still a need in this field for the solvent system of the working solution capable of remarkably improving the production efficiency of the process for producing hydrogen peroxide through anthraquinone process.

## Summary of the Invention

Aiming at the defects of the prior art, an object of the present application is to provide a working solution and a solvent system thereof suitable for the process for producing hydrogen peroxide through anthraquinone process, wherein the solvent system achieves good solubility of both anthraquinone and anthrahydroquinone, low intersolubility with water and stable physicochemical properties, can meet the use requirements of the process according to anthraquinone process, improves the production efficiency of the process for producing hydrogen peroxide through anthraquinone process and has good industrial application prospects.

In order to achieve the above object, in one aspect, the present application provides a solvent system of a working solution for producing hydrogen peroxide through anthraquinone process, which comprises or consists of the following components, in parts by volume:

A) 2-60 parts of an imide derivative represented by formula (I),

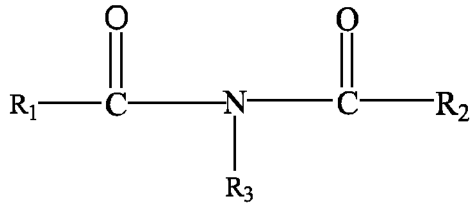

(I),

wherein groups $R_1$, $R_2$ and $R_3$ are each independently selected from $C_{7-10}$ aralkyl, $C_{6-10}$ aryl and $C_{1-10}$ alkyl, and optionally, the groups $R_1$, $R_2$ and $R_3$ are each independently substituted with one or more groups selected from $C_{1-8}$ alkyl, $C_{1-8}$ alkoxy and $C_{1-8}$ acyloxy;

B) 40-98 parts of an aromatic hydrocarbon, preferably $C_{9-10}$ aromatic hydrocarbon;

C) 0-20 parts of trioctyl phosphate; and

D) 0-20 parts of diisobutylcarbinol.

In another aspect, the present application provides a method for preparing the solvent system of the present application, which comprises steps of:

I) providing the imide derivative represented by formula (I) as the component A),

II) mixing the component A) with the component B), the component C) and the component D) according to the parts by volume.

In another aspect, the present application provides a working solution for producing hydrogen peroxide through anthraquinone process, which comprises the solvent system of the present application and a working carrier, wherein the working carrier is selected from one or more of anthraquinone and alkyl substituted derivatives thereof.

In yet another aspect, the present application provides a process for producing hydrogen peroxide through anthraquinone process, comprising steps of hydrogenation, oxidation, extraction and post-treatment of working solution to be recycled, and characterized in that the hydrogenation step is carried out by using the working solution of the present application.

Compared with the existing hydrogen peroxide working solution and solvent system in industry, the novel working solution and solvent system provided by the application have one or more of the following advantages:

1) the working solution obtains good solubility of both anthraquinone and anthrahydroquinone, wherein the solubility of 2-alkyl anthraquinone in the working solution can reach 180 g/L or above under the conditions of normal temperature and normal pressure, and the solubility of 2-alkylanthrahydroquinone in the working solution can respectively reach 99.8 g/L or above under the working condition of anthraquinone process, so that the effective anthraquinone concentration in the working solution can be greatly increased, and the hydrogenation conversion rate of anthraquinone can be increased;

2) the working solution obtains good hydrogen dissolution and mass transfer properties, high hydrogenation efficiency, and low anthraquinone degradation rate, and the hydrogenation efficiency of the working solution can reach 13 g/L or above under the condition of using the anthraquinone hydrogenation catalyst which is conventionally used in industry; and

3) the working solution has low intersolubility with water and low density and viscosity, and the residual carbon value in the hydrogen peroxide product is lower than 200 ppm. Additional features and advantages of the present application will be set forth in detail in the detailed description which follows.

**Detailed description**

The following describes the specific embodiments of the present application in detail. It should be understood that the specific embodiments of the application described therein, are given by way of illustration and explanation only, not limitation.

Any specific numerical value, including the endpoints of a numerical range, described in the context of the present application is not restricted to the exact value thereof, but should be interpreted to further encompass all values close to said exact value, for example all values within ±5% of said exact value. Moreover, regarding any numerical range described herein, arbitrary combinations can be made between the endpoints of the range, between each endpoint and any specific value within the range, or between any two specific values within the range, to provide one or more new numerical range(s), where these new numerical range(s) should also be deemed to have been specifically described in the present application.

Unless otherwise stated, the terms used herein have the same meaning as commonly understood by those skilled in the art; and if the terms are defined herein and their definitions are different from the ordinary understanding in the art, the definition provided herein shall prevail.

In the present application, the term "aromatic hydrocarbon" refers to hydrocarbon derivatives having benzene ring structure(s), and aromatic hydrocarbons include monocyclic aromatic hydrocarbons, polycyclic aromatic hydrocarbons and condensed ring aromatic hydrocarbons, depending on the number and structure of benzene rings contained. As specific examples, the aromatic hydrocarbon includes, but is not limited to, benzene, alkylbenzene, naphthalene, and the like.

In the present application, the term "aryl" refers to a group formed by dehydrogenating one H atom from an aromatic ring of aromatic hydrocarbon. As specific examples, the aryl includes, but is not limited to, phenyl, alkylphenyl, naphthyl, and the like.

In the present application, in addition to those matters explicitly stated, any matter or matters not mentioned are considered to be the same as those known in the art without any change. Moreover, any of the embodiments described herein can be freely combined with another one or more embodiments described herein, and the technical solutions or ideas thus obtained are considered as part of the original disclosure or original description of the present application, and should not be considered to be a new matter that has not been disclosed or anticipated herein, unless it is clear to the person skilled in the art that such a combination is obviously unreasonable.

All of the patent and non-patent documents cited herein, including but not limited to textbooks and journal articles, are hereby incorporated by reference in their entirety.

As described above, in a first aspect, the present application provides a solvent system of a working solution for producing hydrogen peroxide through anthraquinone process, comprising or consisting of following components, in parts by volume:

A) 2-60 parts of an imide derivative represented by formula (I),

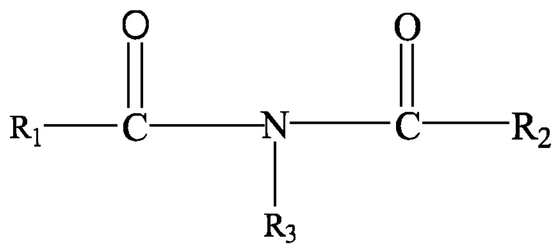

(I),

wherein groups $R_1$, $R_2$ and $R_3$ are each independently selected from $C_{7-10}$ aralkyl, $C_{6-10}$ aryl and $C_{1-10}$ alkyl, and optionally, the groups $R_1$, $R_2$ and $R_3$ are each independently substituted with one or more groups selected from $C_{1-8}$ alkyl, $C_{1-8}$ alkoxy and $C_{1-8}$ acyloxy;
B) 40-98 parts of an aromatic hydrocarbon;
C) 0-20 parts of trioctyl phosphate; and
D) 0-20 parts of diisobutylcarbinol.

In a preferred embodiment, in the imide derivative represented by formula (I), $R_1$, $R_2$ and $R_3$ are each independently selected from $C_{7-8}$ aralkyl, $C_{6-8}$ aryl and $C_{1-8}$ alkyl, and optionally, the groups $R_1$, $R_2$ and $R_3$ are each independently substituted by one or more groups selected from $C_{1-4}$ alkoxy and $C_{2-4}$ acyloxy. As specific examples, the $C_{7-8}$ aralkyl includes, but is not limited to, benzyl and phenethyl; the $C_{6-8}$ aryl includes, but is not limited to, phenyl, methylphenyl, and ethylphenyl; the $C_{1-8}$ alkyl includes, but is not limited to, methyl, ethyl, n-propyl, isopropyl, n-butyl, isobutyl, t-butyl, n-pentyl, isoamyl, heptyl, and octyl; the $C_{1-4}$ alkoxy includes, but is not limited to, methoxy, ethoxy, propoxy, and butoxy; the $C_{2-4}$ acyloxy includes, but is not limited to, acetoxy ($CH_3COO$-) and propionyloxy ($CH_3CH_2COO$-)

In the present application, the number of carbon atoms of the substituent groups at different positions in the molecule of the imide derivative represented by formula (I), i.e., the groups $R_1$ to $R_3$, can be dispensed and adjusted in accordance with the requirements on different physicochemical properties, but the total number of carbon atoms of the groups $R_1$ to $R_3$ is generally not more than 23, preferably not more than 20, and more preferably not more than 18.

In certain preferred embodiments, the solvent system comprises or consists of following components, in parts by volume:

A) 2-60 parts, preferably 5-50 parts, more preferably 15-45 parts, of the imide derivative represented by formula (I); and

B) 40-98 parts, preferably 50-95 parts, more preferably 55-85 parts, of the aromatic hydrocarbon.

In further preferred embodiments, the solvent system comprises or consists of following components, in parts by volume:

A) 2-30 parts, preferably 5-25 parts, of the imide derivative represented by formula (I);
B) 40-96 parts, preferably 55-90 parts, of the aromatic hydrocarbon; and
C) 2-30 parts, preferably 5-20 parts, of trioctyl phosphate.

In other preferred embodiments, the solvent system comprises or consists of following components, in parts by volume:

A) 2-30 parts, preferably 5-20 parts, of the imide derivative represented by formula (I);
B) 50-96 parts, preferably 60-90 parts, of the aromatic hydrocarbon; and
D) 2-20 parts, preferably 5-20 parts, of diisobutylcarbinol.

In certain preferred embodiments, the total volume of the solvent system of the present application is 100 parts.

In the present application, the aromatic hydrocarbon used in component B) is preferably $C_{9-10}$ aromatic hydrocarbon, including but not limited to: mesitylene, pseudocumene, cumene, methylethylbenzene, 2-ethyl-1-methylbenzene and the like. In preferred embodiments, the aromatic hydrocarbon used in component B) is selected from mesitylene, pseudocumene, cumene, methylethylbenzene, 2-ethyl-1-methylbenzene, and any combination thereof.

In a second aspect, there is provided a method for preparing the solvent system according to the first aspect of the present application, comprising steps of:

I) providing the imide derivative represented by formula (I) as the component A),
II) mixing the component A) with the component B), the component C) and the component D) according to the parts by volume.

In a preferred embodiment, said step I) further comprises steps of:

1) subjecting a carboxylic acid represented by formula (II) and a carboxylic acid represented by formula (III) to an intermolecular dehydration reaction to obtain an acid anhydride represented by formula (IV),

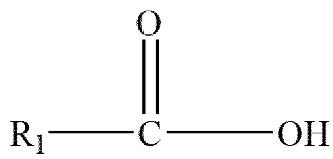

(II)

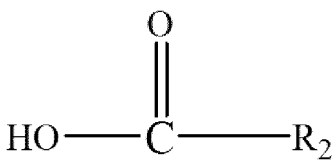

(III)

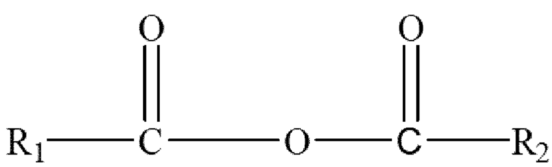

(IV);

2) reacting the acid anhydride represented by formula (IV) with an ammonia source to obtain an imide represented

by formula (V),

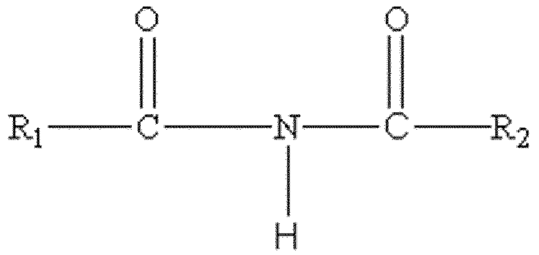

(V);

and

3) subjecting the imide represented by formula (V) and a halogenated hydrocarbon represented by formula (VI) to a nucleophilic substitution reaction,

$R_3$-X (VI),

to obtain the imide derivative represented by formula (I), wherein groups $R_1$, $R_2$ and $R_3$ are as defined above and X represents halogen, preferably chlorine or bromine, more preferably bromine.

In a further preferred embodiment, the dehydration reaction of step 1) is carried out in the presence of a first dehydrating agent selected from $P_2O_5$, potassium carbonate solution, 5A molecular sieve and activated alumina, preferably $P_2O_5$. Taking $P_2O_5$ as the first dehydrating agent as an example, the dehydration reaction formula is as follows:

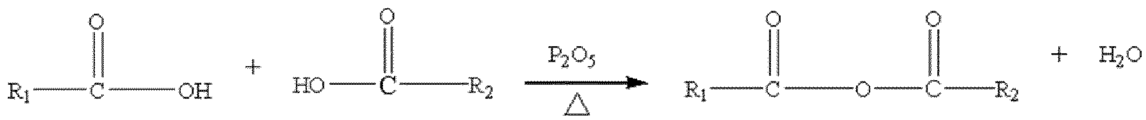


(II) (III) (IV)

.

In a further preferred embodiment, the dehydration reaction of step 1) is carried out in an organic solvent selected from xylene, trimethylbenzene, chlorobenzene, N,N-dimethylformamide, ethyl acetate, pyridine or combinations thereof, preferably the organic solvent is used in an amount of 2-10 mL/g, preferably 3-6 mL/g, relative to the total amount of carboxylic acids.

In a further preferred embodiment, the dehydration reaction of step 1) is carried out in the presence of a first catalyst, which is an aqueous solution of sodium methoxide and iron salt in molar concentrations of 0.01-5 mol/L and 0.1-10 mol/L, respectively.

In a still further preferred embodiment, the dehydration reaction of step 1) is carried out in the presence of the first dehydrating agent and the first catalyst in the organic solvent, and the mole ratio of the carboxylic acid represented by formula (II): the carboxylic acid represented by formula (III): the catalyst: the dehydrating agent is 1: 1: 0.15-0.5: 0.5-5.

In a still further preferred embodiment, the dehydration reaction of step 1) is carried out under conditions including: a reaction pressure of normal pressure, a reaction temperature of 240-280 °C, and a reaction time of 18-25 hours.

In a further preferred embodiment, said step 2) comprises firstly subjecting the acid anhydride represented by formula (IV) to a prehydrolysis reaction at a low temperature of 25-50 °C, and after the reaction substrate is activated in the catalytic system, carrying out an ammonolysis reaction at a high temperature of 210-230 °C. In a further preferred embodiment, said step 2) comprises reacting the acid anhydride represented by formula (IV) with an ammonia source in the presence of a second catalyst and a second dehydrating agent at a low temperature of 25-50 °C for 2-5 hours, and then at a high temperature of 210-230 °C for 1-1.5 hours, wherein the second catalyst is triethylamine, the second dehydrating agent is selected from potassium carbonate, 5A molecular sieve and activated alumina, preferably potassium carbonate, and the two form a homogeneous solution, the molar concentrations of the two in the mixed solution are 0.01-2 mol/L and 0.5-10 mol/L, respectively, and the molar ratio of the two is 1: 0.6-2.5. In this embodiment, taking potassium carbonate as the second dehydrating agent as an example, the ammonolysis reaction formula of the acid anhydride represented by formula (IV) is as follows:

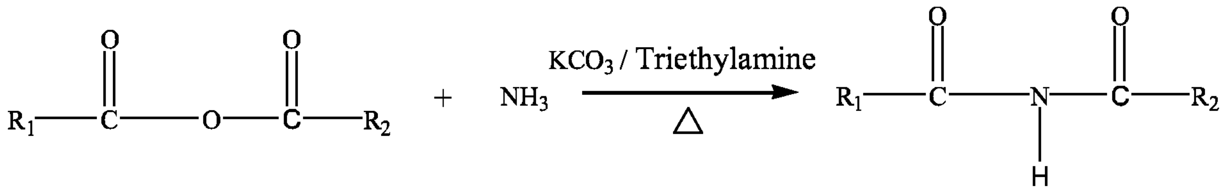


(IV) (V)

In a further preferred embodiment, the ammonia source used in step 2) is selected from ammonia gas, aqueous ammonia, ammonium bicarbonate, urea or combinations thereof, preferably ammonia gas or aqueous ammonia, and the molar ratio of the ammonia source to the acid anhydride represented by formula (IV) is 1.2-10: 1.

In a further preferred embodiment, the reaction pressure of step 2) is 0.1-0.5 MPa.

In a particularly preferred embodiment, the acid anhydride represented by formula (IV) is added to the mixed solution of the second catalyst and the second dehydrating agent by batch feeding or slowly dropwise adding, and the addition time is preferably 25-35 minutes. In a further preferred embodiment, the nucleophilic substitution reaction (β-elimination reaction) of step 3) is carried out under alkaline conditions, so that hydrogen halide produced is dissolved in an alkaline aqueous solution, while the target main product is dissolved in the organic solvent. In a particularly preferred embodiment, the nucleophilic substitution reaction of step 3) is carried out in the reaction medium of step 2), such as a mixed solution of triethylamine and potassium carbonate. According to this embodiment, the reaction formula of the nucleophilic substitution reaction of step 3) is as follows:

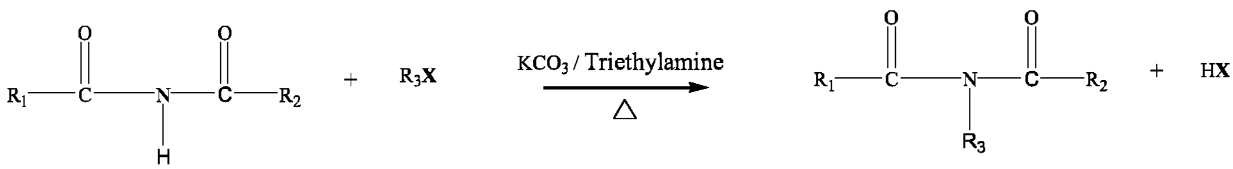


(V) (VI) (I)

In a still further preferred embodiment, the reaction conditions of step 3) include: a reaction temperature of 25-50 °C, and a reaction time of 8-15 hours.

In a still further preferred embodiment, the molar ratio of the halogenated hydrocarbon represented by formula (VI) to the carboxylic acid represented by formula (II) is 1.2-1.5: 1.

In a particularly preferred embodiment, said step I) further comprises steps of:

1) dissolving the carboxylic acid represented by formula (II) and the carboxylic acid represented by formula (III) in an organic solvent, carrying out an intermolecular dehydration reaction in the presence of a first catalyst, a first dehydrating agent at a high temperature, separating the first dehydrating agent after the reaction is completed, and then carrying out extraction and separation to obtain inorganic extract liquor and organic raffinate containing the acid anhydride represented by formula (IV);
2) reacting the organic raffinate containing the acid anhydride represented by formula (IV) obtained in step 1) with an ammonia source under the action of a second catalyst and a second dehydrating agent at a low temperature for a period of time and then at a high temperature for a period of time, and separating an inorganic phase after the reaction is completed to obtain an organic solution containing the imide represented by formula (V);
3) adding a proper amount of the halogenated hydrocarbon represented by formula (VI) into the organic solution containing the imide represented by formula (V), carrying out a nucleophilic substitution reaction under weakly or strongly alkaline conditions, washing and extracting after the reaction is completed to obtain an organic extraction phase, carrying out vacuum distillation to remove the solvent, and drying to obtain the imide derivative product represented by formula (I).

In a further preferred embodiment, said drying in step 3) comprises drying at normal pressure at a temperature of 120-140 °C for 12-24 hours.

In a further preferred embodiment, said washing in step 3) is generally carried out by washing the reaction mixture 2-4 times with deionized water.

In a particularly preferred embodiment, the reaction process of the target product is tracked and monitored by TCL via a layer-by-layer analytical method (ethyl acetate: n-hexane = 2: 1) using silica gel of 300-400 mesh during the synthesis of the imide derivative represented by formula (I). After the reaction is completed, the reaction mixture is

washed for 3 times by using deionized water, then extracted with dichloromethane to obtain an organic extraction phase, subjected to vacuum distillation to remove the solvent, and dried in an oven to obtain the target product as white solid.

The method for synthesizing the imide derivative represented by formula (I) of the present application has the advantages of mild reaction conditions, safety and environmental protection, high reaction yield, low cost, flexible regulation and control on functional groups of imide derivative molecules according to use characteristics and wide application ranges.

In a third aspect, the present application provides a working solution for producing hydrogen peroxide through anthraquinone process, comprising the solvent system of the present application and a working carrier, wherein the working carrier is selected from one or more of anthraquinone and alkyl substituted derivatives thereof, and is preferably 2-alkyl anthraquinone or 2,6-dialkyl anthraquinone.

In a preferred embodiment, the working carrier is a 2-alkyl anthraquinone, preferably selected from 2-ethyl anthraquinone, 2-butyl anthraquinone, 2-amyl anthraquinone or combinations thereof.

In a preferred embodiment, the working carrier has a concentration of at least 10 g/L based on the volume of the working solution and lower than the saturation concentration of the working carrier.

In a further preferred embodiment, the working carrier is 2-ethyl anthraquinone and the concentration of the working carrier is 30-200 g/L, or the working carrier is 2-amyl anthraquinone and the concentration of the working carrier is 10-680 g/L.

In a fourth aspect, the present application provides a process for producing hydrogen peroxide through anthraquinone process, comprising steps of hydrogenation, oxidation, extraction and post-treatment of working solution to be recycled, and characterized in that the hydrogenation step is carried out by using the working solution of the present application.

In some specific embodiments, the process for producing hydrogen peroxide through anthraquinone process of the present application comprises following steps:

1) carrying out a hydrogenation reaction between the working solution of the present application and hydrogen in a hydrogenation reactor filled with a catalyst to obtain a hydrogenated solution containing hydrogenated anthraquinone;
2) carrying out an oxidation reaction on the hydrogenated solution obtained in step 1) in an oxidation reactor under an oxygen-containing atmosphere to obtain an oxidized solution containing hydrogen peroxide;
3) extracting hydrogen peroxide in the oxidized solution with water to obtain an aqueous hydrogen peroxide solution; and
4) after post-treatment procedure, recycling the raffinate obtained after extraction with water.

In a preferred embodiment, the conditions of the hydrogenation step 1) include: a hydrogenation temperature of 25-80 °C, and a pressure of 0.1-0.7 MPa.

In the present application, the reactor type used in the hydrogenation step is not strictly limited, and for example, the hydrogenation reactor may be a fluidized bed, a slurry bed, or a fixed bed.

According to the present application, a hydrogenation catalyst well known in the art can be used in the hydrogenation step, wherein the hydrogenation active component is typically Pd, the carrier is typically alumina or silica gel, and an auxiliary agent component can also be added to the catalyst, such as one or more of Mo, Na, K, Ni, Mg, Au, Ca, Fe, etc. Preferably, based on the weight of the hydrogenation catalyst, the content of the hydrogenation active component in the catalyst is 0.05%-5%, the content of the auxiliary agent is 0.05%-3%, and the balance is the carrier component.

According to the present application, the oxidation step 2) can be carried out using air, pure oxygen or other conventional oxidants, preferably air. The conditions of the oxidation step generally include: an oxidation temperature of 25-70 °C, and a pressure of 0.1-0.5 MPa.

According to the present application, the post-treatment procedure 4) can be carried out by conventional techniques in the art, for example, by dry dehydration and decomposition of $H_2O_2$ via a $K_2CO_3$ solution and separation of the alkali via sedimentation, followed by adsorption of the degradation products and regeneration via activated alumina in a clay bed; alternatively, by partial removal of moisture in the working solution via a vacuum dehydration technology, and 20-50% partial of working solution passing through a $K_2CO_3$ solution, a drying tower and a clay bed respectively to be regenerated in vitro.

## Examples

The present application will be further illustrated in detail with reference to the following examples, but the present application is not limited thereto. In the following examples, reagents and raw materials were used as commercially available products, and the purity was chemically pure, unless otherwise specified.

The present application evaluated the solubility of anthraquinone and anthrahydroquinone in different working

solution systems, as well as the carbon residue index in hydrogen peroxide products, according to the following procedures:

(1) Analysis of anthraquinone solubility: the solubility of anthraquinone was measured by a "solid-liquid dissolution equilibrium method". Under the constant temperature condition of 25 °C, 2-alkyl anthraquinone was gradually dissolved in 200 mL of working solution. After the anthraquinone reached solid-liquid two-phase equilibrium and was not dissolved any more, the working solution was left to stand for 2 hours. After complete layering, 1mL of the supernatant was taken and dissolved in acetonitrile. After dilution by a factor of 200, the concentration of the anthraquinone in the working solution was measured by high performance liquid chromatography. The high performance liquid chromatography test conditions were as follows: Agilent HPLC 1260, 4.6×250mm×5um Eclipse PAH reverse phase chromatographic column, mobile phase: acetonitrile/water = 80/20, ultraviolet detector, detection wavelength: 255 nm, mobile phase flow rate: 1 ml/min, injection volume: 1 ml, chromatographic column temperature: 308.15 K. During the process of measuring the solubility, the temperature accuracy was ± 0.03 °C, and the high performance liquid chromatography analysis error was 0.1 mg. The average value of three parallel experimental data was the experimental value of anthraquinone solubility measured under these conditions.

(2) Analysis of anthrahydroquinone solubility: the solubility of anthrahydroquinone was obtained by a critical hydrogenation experiment. The working solution was subjected to a hydrogenation reaction in a transparent and visualized fixed bed reactor. The flow condition of the working solution in the fixed bed layer was observed by a laser detector. The hydrogenation reaction was stopped when the working solution reached a critical crystallization precipitation state. 1 mL of hydrogenated solution was taken to analyze the composition of the hydrogenated solution by high performance liquid chromatography. Simultaneously, 5 mL of hydrogenated solution was taken to be completely oxidized by air or oxygen, and the mass concentration of hydrogen peroxide in the oxidized solution was analyzed by a potassium permanganate ($KMnO_4$) titration method. And the solubility of anthrahydroquinone in different solvent systems was calculated based on the hydrogenation efficiency value under the condition that there was no degradation product of anthraquinone present in the working solution analyzed by high performance liquid chromatography.

(3) Analysis of carbon residue in hydrogen peroxide: hydrogen peroxide in the oxidized solution was extracted with deionized water, the mixture was left to stand for more than 24 hours. After the extraction phase and the raffinate phase have completely separated, 50 ml of extract liquor was taken at one time, and the content of residual carbon in the extract liquor was analyzed by using a TOC carbon residue analyzer.

(4) Analysis of hydrogen dissolution and mass transfer: the gas-liquid phase equilibrium data of the solvent system for the working solution and hydrogen were measured and analyzed by using a "dissolution saturation method". The analysis device was mainly composed of a 500 ml stainless steel mechanical stirring kettle, a vacuum-pumping system and a pressure and temperature control system. Before measurement, 300 ml of the solvent to be tested was placed in a reaction kettle for vacuum degassing, the airtightness of the measurement system was tested by $N_2$, and if there was no pressure change in the system within 1 hour, it indicated that the the measurement system had good airtightness; the working solution to be tested was heated to a predetermined temperature. After quickly introducing $H_2$ to replace $N_2$ in the device for three times, the stirring in the kettle was started and the change of the hydrogen partial pressure inside the system was recorded as a function of the measurement time simultaneously. The calculation process of the solubility and mass transfer coefficient (*k*a) of hydrogen in different solvent systems was as follows:

$H_2$ inside the constant-volume closed reactor was dissolved and absorbed by the liquid phase main body of the working solution, so that the partial pressure of hydrogen in the reactor was a function of the physical dissolution time. The pressure gradually reduced as a function of the contact time, and the variation relationship thereof is shown as Equation 1:

$$\frac{dP}{dt} = -\frac{RT}{V_G}\frac{dN_L}{dt} \quad \text{(Equation 1)}$$

wherein $N_L$ is the mass transfer rate of $H_2$ in the solvent system, R is the gas constant, P and T are the corresponding pressure and temperature, respectively, under the test conditions, and $V_G$ is the gas phase volume within the test system. Equation 1 is integrated to obtain

$$N_L = \frac{V_G dN_G}{RT\ dt}(p_i - p) \quad \text{(Equation 2).}$$

The above mass equilibrium equation of $H_2$ in the working solution is related to the Henry Equation $P^* = H \cdot C^*$ to obtain

$$\frac{dN_L}{dt} = V_L k_L a \cdot \left(\frac{P}{H} - \frac{N_L}{V_L}\right) \quad \text{(Equation 3)}$$

Equation 4 is obtained by integrating Equation 3 with $\alpha$ calculated as $\alpha = \frac{V_L RT}{V_G H} = \frac{P_i - P_f}{P_f - P_0}$, under the boundary conditions of $P = P_0$, $t = 0$ in the initial state and $P = P_i$, $t = t$ during the mass transfer, according to which the volume transfer coefficient of $H_2$ in the liquid phase main body of the working solution can be calculated,

$$ln\left[\frac{\alpha(P_i - P_f)}{(\alpha + 1)(P_i - P_0) - (P_i - P_f)}\right] = k_L a(\alpha + 1)t \quad \text{(Equation 4).}$$

Preparation Example 1

This preparation example illustrated the synthesis of N-t-butyl di-t-butyl imide represented by the following formula:

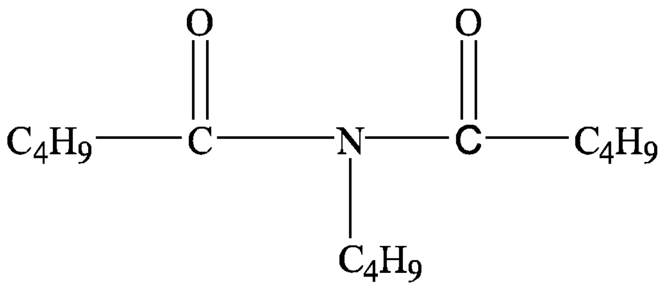

.

176.2 g of tert-valeric acid, 75 g of $P_2O_5$ and 50 mL of a mixed solution of sodium methoxide and iron salt (with 6.8 g of sodium methoxide and 10 g of ferrous nitrate) were charged into a 500 mL reaction kettle at one time. The system was raised to 265 °C in temperature to carry out reaction at constant temperature for 20 hours, after which the reaction was completed. Subsequent to filtering out $P_2O_5$ powder, the reaction solution was transferred to a separatory funnel and washed thoroughly with deionized water for three times. The inorganic aqueous solution was separated; and the organic raffinate was returned back into the reaction kettle. A mixed gas of ammonia and nitrogen was introduced into the reaction system, and the pressure was increased to 0.25-0.3 MPa. The reaction was allowed to proceed for 2 hours at a temperature of 35 °C, during which a mixed solution of 50 mL, 45-50 wt.% of potassium carbonate and triethylamine was pumped into the reaction system for a pumping time of 20 min. Then, the system was raised to 220 °C in temperature to carry out reaction for 1.5 hours. After the system cooled down to room temperature, the inorganic phase in the mixed solution was separated. To the remaining organic phase reaction solution, 165 g of 1-bromobutane was added and allowed to react at 35 °C for 12 hours. The composition of raw materials and products in the reaction system was tracked and monitored by TCL via a layer-by-layer analytical method (ethyl acetate: n-hexane = 2: 1) using silica gel of 300-400 mesh. After the reaction was completed, the reaction product was washed with deionized water for 3 times, and the reaction solution was subjected to vacuum dehydration under the vacuum degree condition of -0.6 to -0.75 MPa. The product was washed with dichloromethane, followed by distilling out the solvent under reduced pressure. The product was then placed in an oven for drying for 4 hours to finally obtain white crystals of N-n-butyl di-t-butyl imide. The obtained product was confirmed by 1H NMR and MS spectrum analysis as N-n-butyl di-t-butyl imide, with a total yield of 95.6% (based on the starting carboxylic acids).

1H NMR (500 MHz, $CDCl_3$) $\delta$ =0.9-1.0 (m, 18H), 1.9-2.05 (m, 1H), 2.05-2.15 (m, 2H), 2.25-2.4 (m, 4H), 3.4-3.45 (s, 2H); MS $[M+H]^+$: 241.7.

Preparation Example 2

This preparation example illustrates the synthesis of N-isoamyl 1-isopropyl-2-t-butyl imide represented by the following formula:

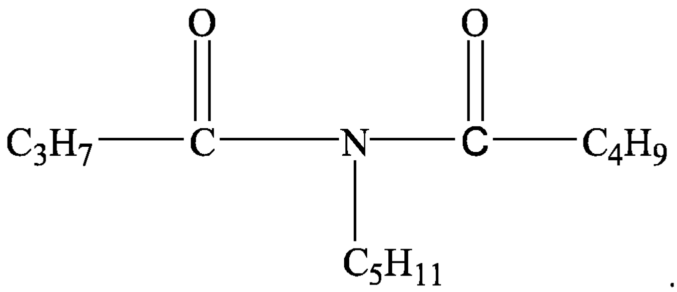

The experiment was carried out with reference to Preparation Example 1, except that the raw materials and the amounts used of the carboxylic acids represented by formulae (II) and (III) and halogenated alkanes were slightly adjusted according to the target product. 88.1 g of tert-valeric acid, 74.5 g of tert-butyric acid, 71 g of $P_2O_5$ and 30 mL of a mixed solution of sodium methoxide and iron salt (with 5.4 g of sodium methoxide and 9.1 g of ferrous nitrate) were respectively charged into a 500 mL reaction kettle at one time. After the intermediate product was obtained by the preparation method as in Preparation Example 1, 165 g of 1-bromopentane was added into the reaction solution of the intermediate product to carry out reaction at 265 °C for 12 hours. The composition of raw materials and products in the reaction system was tracked and monitored by TCL via a layer-by-layer analytical method (ethyl acetate: n-hexane = 2: 1) using silica gel of 300-400 mesh. After the reaction was completed, the reaction product was washed with deionized water for 3 times. The product was washed with dichloromethane, followed by distilling out the solvent under reduced pressure. The product was then placed in an oven for drying for 4 hours to finally obtain the product as white crystals. The obtained product was confirmed to have the structure of N-isoamyl 1-isopropyl-2-t-butyl imide by 1H NMR and MS spectrum analysis, with a yield of 95.6% (based on the starting carboxylic acids)

1H NMR (500 MHz, $CDCl_3$) $\delta$ =0.95-1.10 (m, 18H), 2.07-2.17 (m, 1H), 2.25-2.4 (m, 2H), 2.5-2.55 (m, 2H), 3.45-3.55 (s, 2H);
MS $[M+H]^+$: 241.3.

Preparation Examples 3-5

The experiments were carried out with reference to Preparation Example 1, except that the corresponding carboxylic acids and halogenated hydrocarbon raw materials were selected according to the functional groups ($R_1$, $R_2$, $R_3$) of the different target products.

Specifically, acetic acid, 3-methoxypropionic acid and bromobutane were selected as raw materials in Preparation Example 3; propionic acid, 2-acetoxyacetic acid and bromopentane were selected as raw materials in Preparation Example 4; and acetic acid, 2-methylbenzoic acid and chloropropane were selected as raw materials in Preparation Example 5.

Properties of the products obtained in Preparation Examples 1-5 are shown in Table 1.

TABLE 1. Properties of the products obtained in each of the Preparation Examples

| Example No. | $R_1$ | $R_2$ | $R_3$ | Appearance | Yield | Purity |
|---|---|---|---|---|---|---|
| Preparation Example 1 | $C_4H_9$ | $C_4H_9$ | $C_4H_9$ | White crystals | 96.7 | 99.3% |
| Preparation Example 2 | $C_3H_7$ | $C_4H_9$ | $C_5H_{11}$ | White crystals | 95.6 | 98.2% |
| Preparation Example 3 | $CH_3$ | $CH_3OCH_2CH_2$- | $C_4H_9$ | White crystals | 96.1 | 98.1% |
| Preparation Example 4 | $C_2H_5$ | $CH_3COOCH_2$- | $C_5H_{11}$ | White crystals | 95.9 | 99.5% |
| Preparation Example 5 | $CH_3$ | $CH_3C_6H_5$- | $C_3H_7$ | White crystals | 97.2 | 98.8% |

Example I-1

A mixture of $C_9$-$C_{10}$ aromatic hydrocarbon/N-benzyl di-t-butyl imide = 75/25 by volume fraction was used as a solvent system to prepare a 2-ethyl anthraquinone working solution. The hydrogenation reaction of the above working solution was evaluated by using a 500 ml batch stirred reaction kettle at the hydrogenation temperature of 45-50 °C and the hydrogenation pressure of 0.1-0.3 MPa with the stirring rate of 300-400 rpm; the obtained hydrogenated solution was oxidized with air under conditions of normal pressure and 30-50 °C for 15-30 min; after the oxidized solution was extracted with pure water for 4 times, the hydrogen peroxide content in the extract liquor was measured by the potassium permanganate titration method, and the hydrogenation efficiency of the working solution was calculated; the conventional $Pd/Al_2O_3$ catalyst in hydrogen peroxide industry was used as the catalyst for the hydrogenation experiment, which had a particle diameter of 0.4-0.5 mm, a pore volume of 0.6-0.7 $cm^3/g$, a specific surface area of 150-180 $m^2/g$, and a Pd content of 0.25-0.30 wt.%.

The experimental results showed that: in this working solution solvent system, the solubility of 2-ethyl anthraquinone was 199.5 g/L under the conditions of 25 °C and normal pressure; the solubility of 2-ethyl anthrahydroquinone was 102.5 g/L under the conditions of 50-55 °C and 0.25-0.28 MPa. The hydrogenation efficiency of the working solution with the mass concentration of 2-ethyl anthraquinone being 180 g/L was 14.9 g/L under the conditions of 50-55 °C and 0.25-0.28 MPa, and the organic carbon residue in the hydrogen peroxide product was 155-160 ppm.

Example I-2

A mixture of $C_9$-$C_{10}$ aromatic hydrocarbon/N-phenyl 1-isopropyl-2-t-butyl imide = 75/25 by volume fraction was used as a solvent system to prepare a 2-amyl anthraquinone working solution. The service performance of this working solution system was evaluated by using the process conditions of Example I-1.

The experimental results showed that: in this working solution system, the solubility of 2-amyl anthraquinone was 497.5 g/L under the conditions of 25 °C and normal pressure; the solubility of 2-amyl anthrahydroquinone was 141.9 g/L under the conditions of 60-70 °C and 0.25-0.30 MPa. The hydrogenation efficiency of the working solution with the mass concentration of 2-amyl anthraquinone being 480 g/L was 15.7 g/L under the conditions of 60-70 °C and 0.25-0.30 MPa, and the organic carbon residue in the hydrogen peroxide product was 149.5-154.2 ppm.

Example I-3

A mixture of $C_9$-$C_{10}$ aromatic hydrocarbon/N-isoamyl 1-isopropyl-2-benzyl imide = 80/20 by volume fraction was used as a solvent system to prepare a 2-ethyl anthraquinone working solution. The service performance of this working solution system was evaluated by using the process conditions of Example I-1.

The experimental results showed that: in this working solution system, the solubility of 2-ethyl anthraquinone was 188.3 g/L under the conditions of 25 °C and normal pressure; the solubility of 2-ethyl anthrahydroquinone was 105.8 g/L under the conditions of 53-60 °C and 0.25-0.30 MPa. The hydrogenation efficiency of the working solution with the mass concentration of 2-ethyl anthraquinone being 180 g/L was 13.7 g/L under the conditions of 50-55 °C and 0.25-0.28 MPa, and the organic carbon residue in the hydrogen peroxide product was 145-152 ppm.

Example I-4

A mixture of $C_9$-$C_{10}$ aromatic hydrocarbon/N-ethyl 1-methoxymethyl-2-heptyl imide = 60/40 by volume fraction was used as a solvent system to prepare a 2-ethyl anthraquinone working solution. The service performance of this working solution system was evaluated by using the process conditions of Example I-1.

The experimental results showed that: in this working solution system, the solubility of 2-ethyl anthraquinone was 190.5 g/L under the conditions of 25 °C and normal pressure; the solubility of 2-ethyl anthrahydroquinone was 102.5-103 g/L under the conditions of 50-60 °C and 0.25-0.30 MPa. The hydrogenation efficiency of the working solution with the mass concentration of 2-ethyl anthraquinone being 185 g/L was 14.8 g/L under the conditions of 50-55 °C and 0.25-0.28 MPa, and the organic carbon residue in the hydrogen peroxide product was 150-160 ppm.

Examples I-5

A mixture of $C_9$-$C_{10}$ aromatic hydrocarbon/N-ethyl 1-phenethyl-2-octyl imide = 70/30 by volume fraction was used as a solvent system to prepare a 2-amyl anthraquinone working solution. The service performance of this working solution system was evaluated by using the process conditions of Example I-1.

The experimental results showed that: in this working solution system, the solubility of 2-amyl anthraquinone was 496.5 g/L under the conditions of 25 °C and normal pressure; the solubility of 2-amyl anthrahydroquinone was

135.9-142.5 g/L under the conditions of 60-70 °C and 0.25-0.30 MPa. The hydrogenation efficiency of the working solution with the mass concentration of 2-amyl anthraquinone being 480 g/L was 15.3 g/L under the conditions of 60-70 °C and 0.25-0.30 MPa, and the organic carbon residue in the hydrogen peroxide product was 141-154 ppm.

Comparative Example I-1

A mixture of $C_9$-$C_{10}$ aromatic hydrocarbon/trioctyl phosphate = 75/25 by volume fraction was used as a solvent system to prepare 2-ethyl anthraquinone working solution. The performance of this working solution system was analyzed by using the process conditions of Example I-1.

The experimental results showed that: in the $C_9$-$C_{10}$ aromatic hydrocarbon/trioctyl phosphate working solution system, the solubility of 2-ethyl anthraquinone was 123-130 g/L under the conditions of 25 °C and normal pressure; the solubility of 2-ethyl anthrahydroquinone was 68.5-70 g/L under the conditions of 53-60 °C and 0.25-0.30 MPa. The hydrogenation efficiency of the working solution with the mass concentration of 2-ethyl anthraquinone being 125 g/L was 6.5-7.3 g/L under the conditions of 50-55 °C and 0.25-0.28 MPa, and the organic carbon residue in the hydrogen peroxide product was 205 ppm.

Comparative Example I-2

A working solution was prepared in accordance with Example 3 of CN1552618A. The performance of this working solution system was analyzed by using the process conditions of Example I-1.

The experimental results showed that: under the conditions of 53-60 °C in temperature and 0.25-0.30 MPa in pressure, in the working solution with the mass concentration of 2-ethyl anthraquinone being 154 g/L, the solubility of anthrahydroquinone was 51.5-53.1 g/L, the hydrogenation efficiency was 7.4-7.8 g/L, and the organic carbon residue in the hydrogen peroxide was 351 ppm.

Comparative Example I-3

A 2-ethyl anthraquinone working solution was prepared with the binary solvent system of aromatic hydrocarbon + N-phenyl N-ethyl benzamide (BEA) disclosed in EP0287421. The performance of this working solution system was analyzed by using the process conditions of Example I-1.

The experimental results showed that: in this working solution system, the solubility of 2-ethyl anthraquinone was 155-163 g/L under the conditions of 25 °C and normal pressure; the solubility of 2-ethyl anthrahydroquinone was 85-89 g/L under the conditions of 55-60 °C and 0.25-0.30 MPa. The hydrogenation efficiency of the working solution with the mass concentration of 2-ethyl anthraquinone being 150 g/L was 10.33 g/L under the conditions of 50-55 °C and 0.25-0.28 MPa, and the organic carbon residue in the hydrogen peroxide product was 554.8 ppm.

Example II-1

A mixture of $C_9$-$C_{10}$ aromatic hydrocarbon/trioctyl phosphate/N-t-butyl di-t-butyl imide =75/15/10 by volume fraction was used as a solvent system to prepare the 2-ethyl anthraquinone working solution.

The hydrogenation reaction of the above working solution was evaluated by using a 500 ml batch stirred reaction kettle at the hydrogenation temperature of 45-50 °C and the hydrogenation pressure of 0.1-0.3 MPa with the stirring rate of 300-400 rpm; the obtained hydrogenated solution was oxidized with air under conditions of normal pressure and 30-50 °C for 15-30 min; after the oxidized solution was extracted with pure water for 4 times, the hydrogen peroxide content in the extract liquor was measured by the potassium permanganate titration method, and the hydrogenation efficiency of the working solution was calculated; the conventional $Pd/Al_2O_3$ catalyst in hydrogen peroxide industry was used as the catalyst for the hydrogenation experiment, which had a particle diameter of 0.4-0.5 mm, a pore volume of 0.6-0.7 $cm^3/g$, a specific surface area of 150-180 $m^2/g$, and a Pd content of 0.25-0.30 wt.%.

The experimental results showed that: in this working solution system, the solubility of 2-ethyl anthraquinone was 183.5 g/L under the conditions of 25 °C and normal pressure; the solubility of 2-ethyl anthrahydroquinone was 97.8 g/L under the conditions of 50-55 °C and 0.25-0.28 MPa. The hydrogenation efficiency of the working solution with the mass concentration of 2-ethyl anthraquinone being 180 g/L was 13.2 g/L, the solubility of $H_2$ in this working solution system was 1.45 mmol/L, and the mass transfer factor of $H_2$ was 0.162 $s^{-1}$ under the conditions of 50-55 °C and 0.25-0.28 MPa.

Example II-2

A mixture of $C_9$-$C_{10}$ aromatic hydrocarbon/trioctyl phosphate/N-ethyl 1-methoxymethyl-2-heptyl imide =

60/20/20 by volume fraction was used as a solvent system to prepare a 2-amyl anthraquinone working solution. The service performance of this working solution system was evaluated by using the process conditions of Example II-1.

The experimental results showed that: in this working solution system, the solubility of 2-amyl anthraquinone was 432.1 g/L under the conditions of 25 °C and normal pressure; the solubility of 2-amyl anthrahydroquinone was 145.2 g/L under the conditions of 60-70 °C and 0.25-0.30 MPa. The hydrogenation efficiency of the working solution with the mass concentration of 2-amyl anthraquinone being 430 g/L was 14.5 g/L, the solubility of $H_2$ in this working solution system was 1.56 mmol/L, and the mass transfer factor of $H_2$ was 0.171 $s^{-1}$ under the conditions of 60-70 °C and 0.25-0.30 MPa.

Example II-3

A mixture of $C_9$-$C_{10}$ aromatic hydrocarbon/trioctyl phosphate/N-phenyl 1-isopropyl-2-t-butyl imide = 70/5/25 by volume fraction was used as a solvent system. The service performance of this working solution system was evaluated by using the process conditions of Example II-1.

The experimental results showed that: in this working solution system, the solubility of 2-ethyl anthraquinone was 181.8 g/L under the conditions of 25 °C and normal pressure; the solubility of 2-ethyl anthrahydroquinone was 113.7 g/L under the conditions of 53-60 °C and 0.25-0.30 MPa. The hydrogenation efficiency of the working solution with the mass concentration of 2-ethyl anthraquinone being 180 g/L was 13.7 g/L, the solubility of $H_2$ in this working solution system was 1.49 mmol/L, and the mass transfer factor of $H_2$ was 0.169 $s^{-1}$ under the conditions of 50-55 °C and 0.25-0.28 MPa.

Example II-4

A mixture of $C_9$-$C_{10}$ aromatic hydrocarbon/trioctyl phosphate/N-ethyl-1-phenethyl-2-octyl imide = 80/10/10 by volume fraction was used as a solvent system to prepare a 2-ethyl anthraquinone working solution. The service performance of this working solution system was evaluated by using the process conditions of Example II-1.

The experimental results showed that: in this working solution system, the solubility of 2-ethyl anthraquinone was 192.8 g/L under the conditions of 25 °C and normal pressure; the solubility of 2-ethyl anthrahydroquinone was 99.5 to 102.1 g/L under the conditions of 50-60 °C and 0.25 to 0.30 MPa. The hydrogenation efficiency of the working solution with the mass concentration of 2-ethyl anthraquinone being 190 g/L was 13.6 g/L, the solubility of $H_2$ in this working solution system was 1.52 mmol/L, and the mass transfer factor of $H_2$ was 0.163 $s^{-1}$ under the conditions of 50-55 °C and 0.25-0.28 MPa.

Comparative Example II-1

A mixture of $C_9$-$C_{10}$ aromatic hydrocarbon/trioctyl phosphate = 75/25 by volume fraction was used as a solvent system to prepare 2-ethyl anthraquinone working solution. The performance of this working solution system was analyzed by using the process conditions of Example II-1.

The experimental results showed that: in the $C_9$-$C_{10}$ aromatic hydrocarbon/trioctyl phosphate working solution system, the solubility of 2-ethyl anthraquinone was 123-130 g/L under the conditions of 25 °C and normal pressure; the solubility of 2-ethyl anthrahydroquinone was 68.5-70 g/L under the conditions of 53-60 °C and 0.25-0.30 MPa.

The hydrogenation efficiency of the working solution with the mass concentration of 2-ethyl anthraquinone being 125 g/L was 6.5-7.3 g/L, the solubility of $H_2$ in this working solution system was 1.12 mmol/L, and the mass transfer factor of $H_2$ was 0.125 $s^{-1}$ under the conditions of 50-55 °C and 0.25-0.28 MPa.

Comparative Example II-2

A working solution was prepared in accordance with Example 3 of CN1552618A. The performance of this working solution system was analyzed by using the process conditions of Example II-1.

The experimental results showed that: under the conditions of 53-60 °C in temperature and 0.25-0.30 MPa in pressure, in the working solution with the mass concentration of 2-ethyl anthraquinone being 154 g/L, the solubility of anthrahydroquinone was 51.5-53.1 g/L, the hydrogenation efficiency was 7.4-7.8 g/L, the solubility of $H_2$ in this working solution system was 1.27 mmol/L, and the mass transfer factor of $H_2$ was 0.131 $s^{-1}$.

Comparative Example II-3

A 2-ethyl anthraquinone working solution was prepared with the binary solvent system of aromatic hydrocarbon + N-phenyl N-ethyl benzamide (BEA) disclosed in EP0287421. The performance of this working solution system was

analyzed by using the process conditions of Example II-1.

The experimental results showed that: in this working solution system, the solubility of 2-ethyl anthraquinone was 155-163 g/L under the conditions of 25 °C and normal pressure; the solubility of 2-ethyl anthrahydroquinone was 85-89 g/L under the conditions of 55-60 °C and 0.25-0.30 MPa. The hydrogenation efficiency of the working solution with the mass concentration of 2-ethyl anthraquinone being 150 g/L was 10.33 g/L, the solubility of $H_2$ in this working solution system was 1.30 mmol/L, and the mass transfer factor of $H_2$ was 0.135 $s^{-1}$ under the conditions of 50-55 °C and 0.25-0.28 MPa.

Example III-1

A mixture of $C_9$-$C_{10}$ aromatic hydrocarbon/diisobutylcarbinol/N-t-butyl di-t-butyl imide = 75/5/20 by volume fraction was used as a solvent system to prepare a 2-ethyl anthraquinone working solution.

The viscosity of the working solution was analyzed according to GB 11137-20. The hydrogenation reaction of the above working solution was evaluated by using a 500 ml batch stirred reaction kettle at the hydrogenation temperature of 45-50 °C and the hydrogenation pressure of 0.1-0.3 MPa with the stirring rate of 300-400 rpm; the obtained hydrogenated solution was oxidized with air under conditions of normal pressure and 30-50 °C for 15-30 min; after the oxidized solution was extracted with pure water for 4 times, the hydrogen peroxide content in the extract liquor was measured by the potassium permanganate titration method, and the hydrogenation efficiency of the working solution was calculated; the conventional $Pd/Al_2O_3$ catalyst in hydrogen peroxide industry was used as the catalyst for the hydrogenation experiment, which had a particle diameter of 0.4-0.5 mm, a pore volume of 0.6-0.7 $cm^3/g$, a specific surface area of 150-180 $m^2/g$, and a Pd content of 0.25-0.30 wt.%.

The experimental results showed that: under the condition of 20 °C, the working solution system had a density of 0.917 $g/cm^3$ and a viscosity of 2.331 Pa·s. In this working solution system, the solubility of 2-ethyl anthraquinone was 195.1 g/L under the conditions of 25 °C and normal pressure; the solubility of 2-ethyl anthrahydroquinone was 101.8 g/L under the conditions of 50-55 °C and 0.2-0.3 MPa. The hydrogenation efficiency of the working solution with the mass concentration of 2-ethyl anthraquinone being 180 g/L was 13.6 g/L under the conditions of 50-55 °C and 0.25-0.28 MPa, and the organic carbon residue in the hydrogen peroxide product was 150-160 ppm.

Example III-2

A mixture of $C_9$-$C_{10}$ aromatic hydrocarbon/diisobutylcarbinol/N-isoamyl 1-isopropyl-2-t-butyl imide = 70/15/15 by volume fraction was used as a solvent system to prepare a 2-ethyl anthraquinone working solution. The service performance of this working solution system was evaluated with reference to Example III-1, except that the hydrogenation temperature was 30-40 °C.

The experimental results showed that: under the condition of 20 °C, the working solution system had a density of 0.919 $g/cm^3$ and a viscosity of 2.261 Pa·s. In this working solution system, the solubility of 2-ethyl anthraquinone was 192.2 g/L under the conditions of 25 °C and normal pressure; the solubility of 2-ethyl anthrahydroquinone was 99.8 g/L under the conditions of 30-40 °C and 0.2-0.3 MPa. The hydrogenation efficiency of the working solution with the mass concentration of 2-ethyl anthraquinone being 180 g/L was 13.1 g/L under the conditions of 50-55 °C and 0.25-0.28 MPa, and the organic carbon residue in the hydrogen peroxide product was 140-155 ppm.

Example III-3

A mixture of $C_9$-$C_{10}$ aromatic hydrocarbon/diisobutylcarbinol/N-ethyl 1-methoxymethyl-2-heptyl imide = 60/20/20 by volume fraction was used as a solvent system to prepare a 2-amyl anthraquinone working solution. The service performance of this working solution system was evaluated with reference to Example III-1, except that the hydrogenation temperature was 55-65 °C and the hydrogenation pressure was 0.25-0.40 MPa.

The experimental results showed that: under the condition of 20 °C, the working solution had a density of 0.927 $g/cm^3$ and a viscosity of 2.343 Pa·s. In the working solution system, the solubility of 2-amyl anthraquinone was 477.3 g/L under the conditions of 25 °C and normal pressure; the solubility of 2-amyl anthrahydroquinone was 138.1-145.8 g/L under the conditions of 55-65 °C and 0.25-0.40 MPa. The hydrogenation efficiency of the working solution with the mass concentration of 2-amyl anthraquinone being 460 g/L was 15.1-15.5 g/L under the conditions of 60-70 °C and 0.25-0.30 MPa, and the organic carbon residue in the hydrogen peroxide product was 150-160 ppm.

Comparative Example III-1

A mixture of $C_9$-$C_{10}$ aromatic hydrocarbon/trioctyl phosphate = 75/25 by volume fraction was used as a solvent system to prepare 2-ethyl anthraquinone working solution. The performance of this working solution system was analyzed

by using the process conditions of Example III-1.

The experimental results showed that: under the condition of 20 °C, the working solution had a density of 0.927 g/cm$^3$ and a viscosity of 2.355 Pa·s. In the $C_9$-$C_{10}$ aromatic hydrocarbon/trioctyl phosphate working solution system, the solubility of 2-ethyl anthraquinone was 123-130 g/L under the conditions of 25 °C and normal pressure; the solubility of 2-ethyl anthrahydroquinone was 68.5-70 g/L under the conditions of 53-60 °C and 0.25-0.30 MPa. The hydrogenation efficiency of the working solution with the mass concentration of 2-ethyl anthraquinone being 125 g/L was 6.5-7.3 g/L under the conditions of 50-55 °C and 0.25-0.28 MPa, and the organic carbon residue in the hydrogen peroxide product was 205 ppm.

Comparative Example III-2

A working solution was prepared in accordance with Example 3 of CN1552618A. The performance of this working solution system was analyzed by using the process conditions of Example III-1.

The experimental results showed that: under the condition of 20 °C, the working solution had a density of 0.943 g/cm$^3$ and a viscosity of 2.511 Pa·s. Under the conditions of 53-60 °C in temperature and 0.25-0.30 MPa in pressure, in the working solution with the mass concentration of 2-ethyl anthraquinone being 154 g/L, the solubility of anthrahydroquinone was 51.5-53.1 g/L, the hydrogenation efficiency was 7.4-7.8 g/L, and the organic carbon residue in the hydrogen peroxide was 351 ppm.

Comparative Example III-3

A 2-ethyl anthraquinone working solution was prepared with the binary solvent system of aromatic hydrocarbon + N-phenyl N-ethyl benzamide (BEA) disclosed in EP0287421. The performance of this working solution system was analyzed by using the process conditions of Example III-1.

The experimental results showed that: under the condition of 20 °C, the working solution system had a density of 0.929 g/cm$^3$ and a viscosity of 2.432 Pa·s. In this working solution system, the solubility of 2-ethyl anthraquinone was 155-163 g/L under the conditions of 25 °C and normal pressure; the solubility of 2-ethyl anthrahydroquinone was 85-89 g/L under the conditions of 55-60 °C and 0.25-0.30 MPa. The hydrogenation efficiency of the working solution with the mass concentration of 2-ethyl anthraquinone being 150 g/L was 10.33 g/L under the conditions of 50-55 °C and 0.25-0.28 MPa, and the organic carbon residue in the hydrogen peroxide product was 554.8 ppm.

The present application is illustrated in detail hereinabove with reference to preferred embodiments, but is not intended to be limited to the specific details in the above embodiments. Various simple modifications may be made following the inventive concept of the present application, and these simple modifications shall be within the protection scope of the present application.

In addition, it should be noted that the various technical features described in the above embodiments may be combined in any suitable manner without contradiction, and in order to avoid unnecessary repetition, various possible combinations are not described in the present application.

In addition, the various embodiments of the present application can be arbitrarily combined as long as the combination does not depart from the spirit of the present application, and such combined embodiments should be considered as the disclosure of the present application.

## Claims

1. A solvent system of a working solution for producing hydrogen peroxide through anthraquinone process, comprising or consisting of following components, in parts by volume:

   A) 2-60 parts of an imide derivative represented by formula (I),

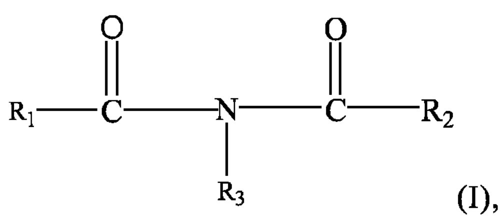

(I),

wherein groups $R_1$, $R_2$ and $R_3$ are each independently selected from $C_{7-10}$ aralkyl, $C_{6-10}$ aryl and $C_{1-10}$ alkyl,

and optionally, the groups $R_1$, $R_2$ and $R_3$ are each independently substituted with one or more groups selected from $C_{1-8}$ alkyl, $C_{1-8}$ alkoxy and $C_{1-8}$ acyloxy;
B) 40-98 parts of an aromatic hydrocarbon, preferably $C_{9-10}$ aromatic hydrocarbon;
C) 0-20 parts of trioctyl phosphate; and
D) 0-20 parts of diisobutylcarbinol.

**2.** The solvent system according to claim 1, wherein in the imide derivative represented by formula (I), $R_1$, $R_2$ and $R_3$ are each independently selected from $C_{7-8}$ aralkyl, $C_{6-8}$ aryl and $C_{1-8}$ alkyl, and optionally, the groups $R_1$, $R_2$ and $R_3$ are each independently substituted by one or more groups selected from $C_{1-4}$ alkoxy and $C_{2-4}$ acyloxy.

**3.** The solvent system according to claim 1 or 2, comprising or consisting of following components, in parts by volume:

A) 2-60 parts, preferably 5-50 parts, of the imide derivative represented by formula (I); and
B) 40-98 parts, preferably 50-95 parts, of the aromatic hydrocarbon, preferably $C_{9-10}$ aromatic hydrocarbon.

**4.** The solvent system according to claim 1 or 2, comprising or consisting of following components, in parts by volume:

A) 2-30 parts, preferably 5-25 parts, of the imide derivative represented by formula (I);
B) 40-96 parts, preferably 55-90 parts, of the aromatic hydrocarbon, preferably $C_{9-10}$ aromatic hydrocarbon; and
C) 2-30 parts, preferably 5-20 parts, of trioctyl phosphate.

**5.** The solvent system according to claim 1 or 2, comprising or consisting of following components, in parts by volume:

A) 2-30 parts, preferably 5-20 parts, of the imide derivative represented by formula (I);
B) 50-96 parts, preferably 60-90 parts, of the aromatic hydrocarbon, preferably $C_{9-10}$ aromatic hydrocarbon; and
D) 2-20 parts, preferably 5-20 parts, of diisobutylcarbinol.

**6.** A method for preparing the solvent system according to any one of claims 1-5, comprising steps of:

I) providing the imide derivative represented by formula (I) as the component A),
II) mixing the component A) with the component B), the component C) and the component D) according to the parts by volume;

preferably, said step I) further comprising steps of:

1) subjecting a carboxylic acid represented by formula (II) and a carboxylic acid represented by formula (III) to an intermolecular dehydration reaction to obtain an acid anhydride represented by formula (IV),

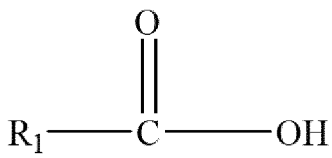

(II)

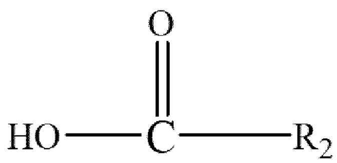

(III)

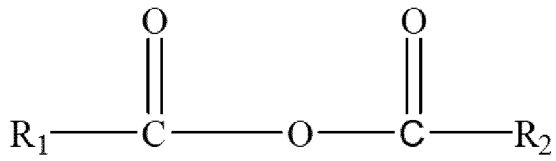

(IV);

2) reacting the acid anhydride represented by formula (IV) with an ammonia source to obtain an imide represented by formula (V),

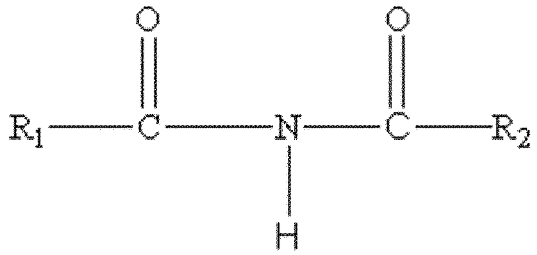

(V);

3) subjecting the imide represented by formula (V) and a halogenated hydrocarbon represented by formula (VI) to a nucleophilic substitution reaction,

$R_3$-X (VI),

to obtain the imide derivative represented by formula (I),
wherein groups $R_1$, $R_2$ and $R_3$ are as defined in claim 1 or 2 and X represents halogen, preferably chlorine or bromine, more preferably bromine.

**7.** The method according to claim 6, **characterized by** one or more of:

the dehydration reaction of step 1) being carried out in the presence of a first dehydrating agent selected from $P_2O_5$, potassium carbonate solution, 5A molecular sieve and activated alumina, preferably $P_2O_5$;
the dehydration reaction of step 1) being carried out in an organic solvent selected from xylene, trimethylbenzene, chlorobenzene, N,N-dimethylformamide, ethyl acetate, pyridine or combinations thereof, preferably the organic solvent being used in an amount of 2-10 mL/g, preferably 3-6 mL/g, relative to the total amount of carboxylic acids; and
the dehydration reaction of step 1) being carried out in the presence of a first catalyst, which is an aqueous solution of sodium methoxide and iron salt in molar concentrations of 0.01-5 mol/L and 0.1-10 mol/L, respectively, preferably, the dehydration reaction of step 1) being carried out in the presence of the first dehydrating agent and the first catalyst in the organic solvent, and the mole ratio of the carboxylic acid represented by formula (II): the carboxylic acid represented by formula (III): the catalyst: the dehydrating agent is 1: 1: 0.15-0.5: 0.5-5; further preferably, the dehydration reaction of step 1) being carried out under conditions including: a reaction pressure of normal pressure, a reaction temperature of 240-280 °C, and a reaction time of 18-25 hours.

**8.** The method according to claim 6 or 7, wherein said step 2) comprises reacting the acid anhydride represented by formula (IV) with an ammonia source in the presence of a second catalyst and a second dehydrating agent at a low temperature of 25-50 °C for 2-5 hours, and then at a high temperature of 210-230 °C for 1-1.5 hours, wherein the second catalyst is triethylamine, the second dehydrating agent is selected from potassium carbonate, 5A molecular sieve and activated alumina, preferably potassium carbonate, and the two form a homogeneous solution, the molar concentrations of the two in the mixed solution are 0.01-2 mol/L and 0.5-10 mol/L, respectively, and the molar ratio of the two is 1: 0.6-2.5; preferably, the ammonia source used in step 2) is selected from ammonia gas, aqueous ammonia, ammonium bicarbonate, urea or combinations thereof, and the molar ratio of the ammonia source to the acid anhydride represented by formula (IV) is 1.2-10: 1;

further preferably, the reaction pressure of step 2) is 0.1-0.5 MPa;

particularly preferably, the acid anhydride represented by formula (IV) is added to the mixed solution of the second catalyst and the second dehydrating agent by batch feeding or slowly dropwise adding for a period of preferably 25-35 minutes.

**9.** The method according to any one of claims 6-8, wherein the nucleophilic substitution reaction of step 3) is carried out under alkaline conditions,

preferably, the reaction conditions of step 3) include: a reaction temperature of 25-50 °C, and a reaction time of 8-15 hours,
further preferably, the molar ratio of the halogenated hydrocarbon represented by formula (VI) to the carboxylic acid represented by formula (II) is 1.2-1.5: 1.

**10.** A working solution for producing hydrogen peroxide through anthraquinone process, comprising the solvent system according to any one of claims 1-5 and a working carrier, wherein the working carrier is selected from one or more of anthraquinone and alkyl substituted derivatives thereof, preferably 2-alkyl anthraquinone or 2,6-dialkyl anthraquinone, more preferably 2-alkyl anthraquinone.

**11.** The working solution according to claim 10, wherein the working carrier is selected from 2-ethyl anthraquinone, 2-butyl anthraquinone, 2-amyl anthraquinone, or combinations thereof.

**12.** The working solution according to claim 10 or 11, wherein the working carrier has a concentration of at least 10 g/L based on the volume of the working solution,
preferably, the working carrier is 2-ethyl anthraquinone and the concentration of the working carrier is 30-200 g/L, or the working carrier is 2-amyl anthraquinone and the concentration of the working carrier is 10-680 g/L.

**13.** A process for producing hydrogen peroxide through anthraquinone process, comprising steps of hydrogenation, oxidation, extraction and post-treatment of working solution to be recycled, and **characterized in that** the hydrogenation step is carried out by using the working solution according to any one of claims 10-12, and preferably, the conditions of the hydrogenation step include: a hydrogenation temperature of 25-80 °C, and a pressure of 0.1-0.7 MPa.

## INTERNATIONAL SEARCH REPORT

International application No.
**PCT/CN2022/123976**

**A. CLASSIFICATION OF SUBJECT MATTER**

C01B 15/023(2006.01)i；C07C 231/02(2006.01)i

According to International Patent Classification (IPC) or to both national classification and IPC

**B. FIELDS SEARCHED**

Minimum documentation searched (classification system followed by classification symbols)

C01B; C07C

Documentation searched other than minimum documentation to the extent that such documents are included in the fields searched

Electronic data base consulted during the international search (name of data base and, where practicable, search terms used)

CNTXT; VEN; WOTXT; USTXT; EPTXT; CNKI; 万方, WANFANG; STN; ISI Web of Science: 中国石油化工股份有限公司, 中石化, 石油化工研究院, 白红鑫, 方向晨, 刘全杰, 贾立明, 蒽醌, 双氧水, 过氧化氢, 工作液, 溶液, 酰亚胺, 芳烃, 磷酸三辛酯, 二异丁基甲醇, anthraquinone, H2O2, hydrogen peroxide, working solution, solvent, +mide, aromatic?, trioctyl phosphate, TOP, diisobutylcarbinol, 式(I)结构, structure of formula (I)

**C. DOCUMENTS CONSIDERED TO BE RELEVANT**

| Category* | Citation of document, with indication, where appropriate, of the relevant passages | Relevant to claim No. |
|---|---|---|
| X | CN 103880699 A (LI NA) 25 June 2014 (2014-06-25)<br>description, paragraphs [0038]-[0050] | 1-3, |
| X | LI, Guangzhe et al. "Rhodium-Catalyzed Synthesis and Reactions of N-Acylphthalimides"<br>*Asian Journal of Organic Chemistry,* Vol. 2, No. 11, 15 July 2013 (2013-07-15),<br>ISSN: 2193-5815,<br>page 985, Scheme 6, and Supporting Information, page 4 | 1-3, |
| X | GARDUNO-CASTRO M. H. et al. "Application of Acyclic Chiral Auxiliaries on Alkylation Reactions"<br>*Tetrahedron Letters,* Vol. 55, No. 1, 07 November 2013 (2013-11-07),<br>ISSN: 0040-4039,<br>page 194, Scheme 2, and Supporting Information, page 3 | 1-3 |
| Y | CN 103880699 A (LI NA) 25 June 2014 (2014-06-25)<br>description, paragraphs [0038]-[0050] | 6-9 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 December 2022** | **30 December 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/123976**

| C. DOCUMENTS CONSIDERED TO BE RELEVANT | | |
|---|---|---|
| Category* | Citation of document, with indication, where appropriate, of the relevant passages | Relevant to claim No. |
| Y | LI, Guangzhe et al. "Rhodium-Catalyzed Synthesis and Reactions of N-Acylphthalimides" *Asian Journal of Organic Chemistry*, Vol. 2, No. 11, 15 July 2013 (2013-07-15), ISSN: 2193-5815, page 985, Scheme 6, and Supporting Information, page 4 | 6-9 |
| Y | GARDUNO-CASTRO M. H. et al. "Application of Acyclic Chiral Auxiliaries on Alkylation Reactions" *Tetrahedron Letters*, Vol. 55, No. 1, 07 November 2013 (2013-11-07), ISSN: 0040-4039, page 194, Scheme 2, and Supporting Information, page 3 | 6-9 |
| Y | CN 101891644 A (EAST CHINA UNIVERSITY OF SCIENCE AND TECHNOLOGY) 24 November 2010 (2010-11-24) description, paragraphs [0011]-[0015] | 6-9 |
| A | CN 1552618 A (SUPENG INDUSTRIAL CO., LTD., SHANGHAI) 08 December 2004 (2004-12-08) entire document | 1-13 |
| A | CN 1583546 A (LIMING CHEMICAL INDUSTRY ACADEMY) 23 February 2005 (2005-02-23) entire document | 1-13 |
| A | CN 111071993 A (CHINA PETROLEUM & CHEMICAL CORP. et al.) 28 April 2020 (2020-04-28) entire document | 1-13 |
| A | US 4803063 A (ATOCHEM et al.) 07 February 1989 (1989-02-07) entire document | 1-13 |
| A | CN 103880699 A (LI NA) 25 June 2014 (2014-06-25) description, paragraphs [0038]-[0050] | 4, 5, 10-13 |
| A | LI, Guangzhe et al. "Rhodium-Catalyzed Synthesis and Reactions of N-Acylphthalimides" *Asian Journal of Organic Chemistry*, Vol. 2, No. 11, 15 July 2013 (2013-07-15), ISSN: 2193-5815, page 985, Scheme 6, and Supporting Information, page 4 | 4, 5, 10-13 |
| A | GARDUNO-CASTRO M. H. et al. "Application of Acyclic Chiral Auxiliaries on Alkylation Reactions" *Tetrahedron Letters*, Vol. 55, No. 1, 07 November 2013 (2013-11-07), ISSN: 0040-4039, page 194, Scheme 2, and Supporting Information, page 3 | 4, 5, 10-13 |

Form PCT/ISA/210 (second sheet) (January 2015)

**INTERNATIONAL SEARCH REPORT**
**Information on patent family members**

International application No.
**PCT/CN2022/123976**

| Patent document cited in search report | | | Publication date (day/month/year) | Patent family member(s) | | | Publication date (day/month/year) |
|---|---|---|---|---|---|---|---|
| CN | 103880699 | A | 25 June 2014 | CN | 103880699 | B | 08 April 2015 |
| CN | 101891644 | A | 24 November 2010 | None | | | |
| CN | 1552618 | A | 08 December 2004 | CN | 1216797 | C | 31 August 2005 |
| CN | 1583546 | A | 23 February 2005 | None | | | |
| CN | 111071993 | A | 28 April 2020 | CN | 111071993 | B | 04 June 2021 |
| US | 4803063 | A | 07 February 1989 | FR | 2614015 | A1 | 21 October 1988 |
| | | | | DE | 3863117 | D1 | 11 July 1991 |
| | | | | EP | 0287421 | A1 | 19 October 1988 |
| | | | | JP | S63274601 | A | 11 November 1988 |
| | | | | AU | 1407288 | A | 20 October 1988 |
| | | | | DK | 168282 | B1 | 07 March 1994 |
| | | | | CA | 1311601 | C | 22 December 1992 |
| | | | | BR | 8801835 | A | 22 November 1988 |
| | | | | FI | 881775 | A | 17 October 1988 |
| | | | | ES | 2022650 | B | 01 December 1991 |
| | | | | DK | 206288 | A | 17 October 1988 |
| | | | | NO | 881630 | A | 07 November 1988 |
| | | | | EP | 0287421 | B1 | 05 June 1991 |
| | | | | KR | 960002618 | B1 | 24 February 1996 |
| | | | | PT | 87238 | A | 12 May 1989 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 1552618 A
- CN 1583546 A
- EP 0287421 A
- CN 111071993 A
- GB 1113720 A